# EUROPEAN PATENT SPECIFICATION

(11) **EP 4 321 864 B1**
(45) Date of publication and mention of the grant of the patent: **08.04.2026**
(21) Application number: 22785020.3
(22) Date of filing: 08.04.2022
(51) Int. Cl.: G01N 33/574, C12Q 1/6886

(54) **COMPOSITION AND KIT FOR DIAGNOSING CANCER AND METHOD FOR DIAGNOSING CANCER USING SAME**
ZUSAMMENSETZUNG UND KIT ZUR DIAGNOSE VON KREBS UND VERFAHREN ZUR DIAGNOSE VON KREBS DAMIT
COMPOSITION ET TROUSSE POUR LE DIAGNOSTIC DU CANCER ET PROCÉDÉ DE DIAGNOSTIC DU CANCER UTILISANT UNE TELLE TROUSSE

(30) Priority: 09.04.2021 KR 20210046621
(43) Date of publication of application: 14.02.2024
(73) Proprietor: Attis Lab, Daejeon 34121 (KR)
(72) Inventor: BHANG, Dong Ha, Seoul 07002 (KR)
(74) Representative: HGF
(86) International application number: PCT/KR2022/005127
(87) International publication number: WO 2022/216111

(56) References cited:
- WO-A1-2005/088312
- WO-A1-2018/069822
- WO-A1-2019/048588
- CN-A- 103 874 770
- KR-A- 20180 103 024
- KR-A- 20190 009 004
- KR-B1- 101 783 994
- US-A1- 2009 123 948
- NESTEROVA M ET AL: "Autoantibody biomarker opens a new gateway for cancer diagnosis", BIOCHIMICA ET BIOPHYSICA ACTA. MOLECULAR BASIS OF DISEASE, AMSTERDAM, NL, vol. 1762, no. 4, 1 April 2006 (2006-04-01), pages 398 - 403, XP027998163, ISSN: 0925-4439, [retrieved on 20060401], DOI: 10.1016/J.BBADIS.2005.12.010
- PAWEL ZAYAKIN ET AL: "Tumor-associated autoantibody signature for the early detection of gastric cancer", INTERNATIONAL JOURNAL OF CANCER, JOHN WILEY & SONS, INC, US, vol. 132, no. 1, 3 July 2012 (2012-07-03), pages 137 - 147, XP071287362, ISSN: 0020-7136, DOI: 10.1002/IJC.27667
- WANG SHUAIBING ET AL: "Using a panel of multiple tumor-associated antigens to enhance autoantibody detection for immunodiagnosis of gastric cancer", ONCOIMMUNOLOGY, 27 March 2018 (2018-03-27), pages e1452582, XP093191385, ISSN: 2162-402X, DOI: 10.1080/2162402X.2018.1452582
- WERNER SIMONE ET AL: "Evaluation of the diagnostic value of 64 simultaneously measured autoantibodies for early detection of gastric cancer", vol. 6, no. 1, 3 May 2016 (2016-05-03), US, XP093191390, ISSN: 2045-2322, Retrieved from the Internet <URL:https://www.nature.com/articles/srep25467.pdf> DOI: 10.1038/srep25467
- WERNER SIMONE ET AL: "Supplemental information", 3 May 2016 (2016-05-03), XP093191512, Retrieved from the Internet <URL:https://www.nature.com/articles/srep25467#Sec14>
- CUI CHI ET AL: "Identification of Novel Autoantibodies Based on the Human Proteomic Chips and Evaluation of Their Performance in the Detection of Gastric Cancer", FRONTIERS IN ONCOLOGY, vol. 11, 26 February 2021 (2021-02-26), XP093191388, ISSN: 2234-943X, DOI: 10.3389/fonc.2021.637871
- MING-MING TSAI: "Potential prognostic, diagnostic and therapeutic markers for human gastric cancer", WORLD JOURNAL OF GASTROENTEROLOGY, vol. 20, no. 38, 1 January 2014 (2014-01-01), pages 13791, XP055178035, ISSN: 1007-9327, DOI: 10.3748/wjg.v20.i38.13791
- NING SHUFANG ET AL: "Clinical significance and diagnostic capacity of serum TK1, CEA, CA 19-9 and CA 72-4 levels in gastric and colorectal cancer patients", JOURNAL OF CANCER, vol. 9, no. 3, 1 January 2018 (2018-01-01), AU, pages 494 - 501, XP093191460, ISSN: 1837-9664, DOI: 10.7150/jca.21562
- WANG HUI-JU ET AL: "Expressions of Neutrophil Gelatinase-Associated Lipocalin in Gastric Cancer: A Potential Biomarker for Prognosis and an Ancillary Diagnostic Test", THE ANATOMICAL RECORD, vol. 293, no. 11, 20 August 2010 (2010-08-20), Hoboken, NJ : Wiley-Liss, pages 1855 - 1863, XP093191531, ISSN: 1932-8486, DOI: 10.1002/ar.21230

## Description

### Technical Field

The present invention relates to a composition and a kit for diagnosing cancer, and a method for diagnosing cancer or a method for detecting a biomarker for diagnosing cancer, using the same.

### Background Art

The World Health Organization (WHO) said that one-third of cancers can be cured through early screening and early treatment. The causes of cancer are diverse, but the number of causes due not only to genetic causes but also to the external environment is increasing. The causes of diseases caused by the external environment are so diverse that it is difficult to explain them with a single cause. Diseases caused by external environmental causes cannot be predicted and prevented in advance, and treatment is carried out only after the disease is diagnosed, so there are problems with cost, period, and considerable time required for recovery. Therefore, in order to diagnose cancer at an early stage, there is a need to develop a biomarker or a combination of biomarkers that can enhance the accuracy, sensitivity, and specificity for cancer diagnosis.

In addition, as the market for companion animals grows, there is a need to develop diagnosis technology for tumors, which are the number one cause of death in companion animals. Due to the high cost barrier of conventional diagnosis of malignant tumor and the lack of prompt diagnosis, the golden time for treatment can be missed. As a technology for point-of-care testing-type diagnosis in a companion dog disease, VDI's VDI-TKCANINE+, Sentinel Biomedical's CADET, VBTA (Veterinary Bladder Tumor Antigen) test method, Alertix Veterinary Diagnostics AB's Alertix Canine, and the like are commercially available. In Korea, a method for detecting cancer in dog has been developed in Korean Patent Application No. 10-2019-7001786 (October 10, 2017).

S. Wemer et al (2016) discloses a signature panel for early diagnosis of gastric cancer which includes autoantibodies against p53 and autoantibodies against Her2.

Therefore, there is a need to develop a simple, rapid and accurate cancer diagnosis technology by developing a cancer biomarker or multiple biomarkers.

### Detailed Description of Invention

### Technical Problem

There is provided a composition for gastric cancer

There is provided a kit for gastric cancer.

There is provided a method for diagnosing gastric cancer.

### Solution to Problem

The invention is as described in the claims.

In one aspect, there is provided a composition for diagnosing gastric as defined in claims **1-8.**

Disclosed but not part of the invention, there is provided a composition for diagnosing bladder cancer, comprising:
an agent for measuring the expression level of a gene or the expression level of a protein or a fragment thereof for a combination of anti-ECPKA antibody, anti-TK1 antibody, anti-granulin antibody, and anti-Her-2 antibody; and one or more biomarkers selected from the group consisting of canine TK1 (cTK1), CEA, AFP, and CRP.

The biomarker combination may further comprise one or more biomarkers selected from the group consisting of anti-human P53 (hP53) antibody, anti-c-myc antibody, human PKA (hPKA), PSA, B2M, and NGAL.

The biomarker combination may comprise a biomarker combination selected from the group consisting of:
(i) anti-ECPKA antibody, anti-TK1 antibody, anti-granulin antibody, anti-Her-2 antibody, anti-hP53 antibody, anti-c-myc antibody, cTK1, AFP, CEA, CRP, B2M, and NGAL;
(ii) anti-ECPKA antibody, anti-TK1 antibody, anti-granulin antibody, anti-Her-2 antibody, anti-hP53 antibody, anti-c-myc antibody, cTK1, AFP, CEA, PSA, CRP, and B2M; and
(iii) anti-ECPKA antibody, anti-TK1 antibody, anti-granulin antibody, anti-Her-2 antibody, anti-hP53 antibody, anti-c-myc antibody, cTK1, AFP, CEA, PSA, CRP, B2M, and NGAL.

The biomarker combination may further comprise a biomarker combination selected from the group consisting of:
(i) anti-ECPKA antibody, anti-TK1 antibody, anti-granulin antibody, anti-Her-2 antibody, anti-hP53 antibody, anti-c-myc antibody, cP53, hPKA, cTK1, AFP, CEA, PSA, CRP, and B2M;
(ii) anti-ECPKA antibody, anti-TK1 antibody, anti-granulin antibody, anti-Her-2 antibody, anti-hP53 antibody, anti-c-myc antibody, cP53, hPKA, cTK1, AFP, CEA, PSA, CRP, B2M, and NGAL;
(iii) anti-ECPKA antibody, anti-TK1 antibody, anti-granulin antibody, anti-Her-2 antibody, anti-hP53 antibody, anti-c-myc antibody, cTK1, AFP, and CRP;
(iv) anti-ECPKA antibody, anti-TK1 antibody, anti-granulin antibody, anti-Her-2 antibody, anti-hP53 antibody, anti-c-myc antibody, cTK1, AFP, and CEA;
(v) anti-ECPKA antibody, anti-TK1 antibody, anti-granulin antibody, anti-Her-2 antibody, anti-hP53 antibody, anti-c-myc antibody, cTK1, AFP, CEA, and PSA; and
(vi) anti-ECPKA antibody, anti-TK1 antibody, anti-granulin antibody, anti-Her-2 antibody, anti-hP53 antibody, anti-c-myc antibody, cTK1, AFP, CEA, PSA, and CRP.

The biomarker combination may further comprise a biomarker combination selected from the group consisting of:
(i) anti-ECPKA antibody, anti-TK1 antibody, anti-granulin antibody, and anti-Her-2 antibody;
(ii) anti-ECPKA antibody, anti-TK1 antibody, anti-granulin antibody, anti-Her-2 antibody, anti-hP53 antibody, and anti-c-myc antibody;
(iii) anti-ECPKA antibody, anti-TK1 antibody, anti-granulin antibody, anti-Her-2 antibody, anti-hP53 antibody, anti-c-myc antibody, and cP53;
(iv) anti-ECPKA antibody, anti-TK1 antibody, anti-granulin antibody, anti-Her-2 antibody, anti-hP53 antibody, anti-c-myc antibody, cP53, hPKA, and cTK1;
(v) anti-ECPKA antibody, anti-TK1 antibody, anti-granulin antibody, anti-Her-2 antibody, anti-hP53 antibody, anti-c-myc antibody, cP53, hPKA, cTK1, and AFP;
(vi) anti-ECPKA antibody, anti-TK1 antibody, anti-granulin antibody, anti-Her-2 antibody, anti-hP53 antibody, anti-c-myc antibody, cP53, hPKA, cTK1, AFP, and CEA;
(vii) anti-ECPKA antibody, anti-TK1 antibody, anti-granulin antibody, anti-Her-2 antibody, anti-hP53 antibody, anti-c-myc antibody, cP53, hPKA, cTK1, AFP, CEA, and PSA;
(viii) anti-ECPKA antibody, anti-TK1 antibody, anti-granulin antibody, anti-Her-2 antibody, anti-hP53 antibody, anti-c-myc antibody, cP53, hPKA, cTK1, AFP, CEA, PSA, and CRP;
(ix) anti-ECPKA antibody, anti-TK1 antibody, anti-granulin antibody, anti-Her-2 antibody, anti-hP53 antibody, and anti-c-myc antibody;
(x) anti-ECPKA antibody, anti-TK1 antibody, anti-granulin antibody, anti-Her-2 antibody, anti-hP53 antibody, anti-c-myc antibody, and cTK1; and
(xi) anti-ECPKA antibody, anti-TK1 antibody, anti-granulin antibody, anti-Her-2 antibody, anti-hP53 antibody, anti-c-myc antibody, cTK1, and AFP.

Also disclosed but not part of the invention, there is provided a composition for diagnosing breast cancer, comprising:
an agent for measuring the expression level of a gene or the expression level of a protein or a fragment thereof for a combination of anti-ECPKA antibody, anti-TK1 antibody, anti-granulin antibody, and anti-Her-2 antibody; and one or more biomarkers selected from the group consisting of cTK1, AFP, B2M, and NGAL.

The biomarker combination may further comprise one or more biomarkers selected from the group consisting of anti-hP53 antibody, anti-c-myc antibody, cP53, hPKA, CEA, and CRP.

The biomarker combination may comprise the following biomarker combination:
anti-ECPKA antibody, anti-TK1 antibody, anti-granulin antibody, anti-Her-2 antibody, anti-hP53 antibody, anti-c-myc antibody, cP53, hPKA, cTK1, AFP, CEA, PSA, CRP, B2M, and NGAL.

The biomarker combination may further comprise the following biomarker combination:
anti-ECPKA antibody, anti-TK1 antibody, anti-granulin antibody, anti-Her-2 antibody, anti-hP53 antibody, anti-c-myc antibody, cP53, hPKA, cTK1, AFP, CEA, CRP, B2M, and NGAL.

The biomarker combination may further comprise a biomarker combination selected from the group consisting of:
(i) anti-ECPKA antibody, anti-TK1 antibody, anti-granulin antibody, anti-Her-2 antibody, anti-hP53 antibody, anti-c-myc antibody, cTK1, AFP, CEA, CRP, B2M, and NGAL;
(ii) anti-granulin antibody, anti-Her-2 antibody, anti-hP53 antibody, anti-c-myc antibody, cP53, hPKA, cTK1, AFP, CEA, PSA, CRP, B2M, and NGAL;
(iii) anti-ECPKA antibody, anti-TK1 antibody, anti-granulin antibody, anti-Her-2 antibody, anti-hP53 antibody, anti-c-myc antibody, cTK1, AFP, CEA, PSA, CRP, B2M, and NGAL;
(iv) anti-ECPKA antibody, anti-TK1 antibody, anti-granulin antibody, anti-Her-2 antibody, TK1, AFP, B2M, and NGAL;
(v) anti-ECPKA antibody, anti-TK1 antibody, anti-granulin antibody, anti-Her-2 antibody, anti-hP53 antibody, anti-c-myc antibody, cP53, cTK1, AFP, CEA, CRP, B2M, and NGAL; and
(vi) anti-ECPKA antibody, anti-TK1 antibody, anti-granulin antibody, anti-Her-2 antibody, anti-hP53 antibody, anti-c-myc antibody, hPKA, cTK1, AFP, CEA, CRP, B2M, and NGAL.

Also disclosed but not part of the invention, there is provided a composition for diagnosing cervical cancer, comprising:
an agent for measuring the expression level of a gene or the expression level of a protein or a fragment thereof for a combination of anti-ECPKA antibody, anti-TK1 antibody, anti-granulin antibody, and anti-Her-2 antibody; and one or more biomarkers selected from the group consisting of AFP and B2M.

The biomarker combination may further comprise one or more biomarkers selected from the group consisting of anti-hP53 antibody, anti-c-myc antibody, hPKA, cTK1, CEA, PSA, CRP, and NGAL.

The biomarker combination may comprise a biomarker combination selected from the group consisting of:
(i) anti-ECPKA antibody, anti-TK1 antibody, anti-granulin antibody, anti-Her-2 antibody, anti-hP53 antibody, anti-c-myc antibody, cP53, hPKA, cTK1, AFP, CEA, PSA, CRP, and B2M;
(i) anti-ECPKA antibody, anti-TK1 antibody, anti-granulin antibody, anti-Her-2 antibody, anti-hP53 antibody, anti-c-myc antibody, cTK1, AFP, CEA, CRP, B2M, and NGAL;
(iii) anti-ECPKA antibody, anti-TK1 antibody, anti-granulin antibody, anti-Her-2 antibody, anti-hP53 antibody, anti-c-myc antibody, cTK1, AFP, CEA, PSA, CRP, and B2M;
(iv) anti-ECPKA antibody, anti-TK1 antibody, anti-granulin antibody, anti-Her-2 antibody, anti-hP53 antibody, anti-c-myc antibody, cTK1, AFP, CEA, PSA, CRP, B2M, and NGAL; and
(v) anti-ECPKA antibody, anti-TK1 antibody, anti-granulin antibody, anti-Her-2 antibody, AFP, and B2M.

The biomarker combination may further comprise a biomarker combination selected from the group consisting of:
(i) anti-ECPKA antibody, anti-TK1 antibody, anti-granulin antibody, anti-Her-2 antibody, anti-hP53 antibody, anti-c-myc antibody, cP53, hPKA, cTK1, AFP, CEA, PSA, CRP, B2M, and NGAL;
(ii) anti-granulin antibody, anti-Her-2 antibody, anti-hP53 antibody, anti-c-myc antibody, cP53, hPKA, cTK1, AFP, CEA, PSA, CRP, B2M, and NGAL;
(iii) anti-granulin antibody, anti-Her-2 antibody, anti-hP53 antibody, anti-c-myc antibody, cTK1, AFP, CEA, PSA, CRP, B2M, and NGAL; and
(iv) anti-ECPKA antibody, anti-TK1 antibody, AFP, and B2M.

The biomarker combination may further comprise the biomarker combination of anti-ECPKA antibody, anti-TK1 antibody, and B2M.

Also disclosed but not part of the invention, there is provided a composition for diagnosing colorectal cancer, comprising:
an agent for measuring the expression level of a gene or the expression level of a protein or a fragment thereof for a combination of anti-ECPKA antibody, anti-TK1 antibody, anti-granulin antibody, and anti-Her-2 antibody; and one or more biomarkers selected from the group consisting of AFP, CEA, CRP, and NGAL.

The biomarker combination may further comprise one or more biomarkers selected from the group consisting of anti-hP53 antibody, anti-c-myc antibody, cTK1, PSA, and B2M.

The biomarker combination may comprise the biomarker combination of anti-ECPKA antibody, anti-TK1 antibody, anti-granulin antibody, anti-Her-2 antibody, AFP, CEA, CRP, and NGAL.

The biomarker combination may further comprise the biomarker combination of anti-ECPKA antibody, anti-TK1 antibody, anti-granulin antibody, anti-Her-2 antibody, anti-hP53 antibody, anti-c-myc antibody, cTK1, AFP, CEA, PSA, CRP, B2M, and NGAL.

The biomarker combination may further comprise a biomarker combination selected from the group consisting of:
(i) anti-ECPKA antibody, anti-TK1 antibody, anti-granulin antibody, anti-Her-2 antibody, anti-hP53 antibody, anti-c-myc antibody, cP53, hPKA, cTK1, AFP, CEA, PSA, CRP, B2M, and NGAL;
(ii) anti-ECPKA antibody, anti-TK1 antibody, anti-granulin antibody, anti-Her-2 antibody, anti-hP53 antibody, anti-c-myc antibody, cTK1, AFP, CEA, CRP, B2M, and NGAL; and
(iii) anti-ECPKA antibody, anti-TK1 antibody, anti-granulin antibody, anti-Her-2 antibody, AFP, and CEA.

Also disclosed but not part of the invention, there is provided a composition for diagnosing liver cancer, comprising:
an agent for measuring the expression level of a gene or the expression level of a protein or a fragment thereof for a combination of anti-ECPKA antibody, anti-TK1 antibody, anti-granulin antibody, and anti-Her-2 antibody; and one or more biomarkers selected from the group consisting of anti-hP53 antibody, anti-c-myc antibody, AFP, and cTK1.

The biomarker combination may further comprise one or more biomarkers selected from the group consisting of cP53, hPKA, CEA, PSA, and CRP.

The biomarker combination may comprise the biomarker combination of anti-ECPKA antibody, anti-TK1 antibody, anti-granulin antibody, anti-Her-2 antibody, anti-hP53 antibody, anti-c-myc antibody, cP53, hPKA, cTK1, AFP, CEA, PSA, and CRP.

The biomarker combination may further comprise a biomarker combination selected from the group consisting of:
(i) anti-ECPKA antibody, anti-TK1 antibody, anti-granulin antibody, anti-Her-2 antibody, anti-hP53 antibody, anti-c-myc antibody, cP53, hPKA, cTK1, and AFP;
(ii) anti-ECPKA antibody, anti-TK1 antibody, anti-granulin antibody, anti-Her-2 antibody, anti-hP53 antibody, anti-c-myc antibody, cP53, hPKA, cTK1, AFP, and CEA;
(iii) anti-ECPKA antibody, anti-TK1 antibody, anti-granulin antibody, anti-Her-2 antibody, anti-hP53 antibody, anti-c-myc antibody, cP53, hPKA, cTK1, AFP, CEA, and PSA;
(iv) anti-ECPKA antibody, anti-TK1 antibody, anti-granulin antibody, anti-Her-2 antibody, anti-hP53 antibody, anti-c-myc antibody, cP53, hPKA, cTK1, AFP, CEA, PSA, CRP, B2M, and NGAL;
(v) anti-ECPKA antibody, anti-TK1 antibody, anti-granulin antibody, anti-Her-2 antibody, anti-hP53 antibody, anti-c-myc antibody, cTK1, AFP, CEA, CRP, B2M, and NGAL;
(vi) anti-ECPKA antibody, anti-TK1 antibody, anti-granulin antibody, anti-Her-2 antibody, anti-hP53 antibody, anti-c-myc antibody, cTK1, AFP, CEA, PSA, CRP, B2M, and NGAL; and
(vii) anti-ECPKA antibody, anti-TK1 antibody, anti-granulin antibody, anti-Her-2 antibody, anti-hP53 antibody, anti-c-myc antibody, cTK1, AFP, CEA, CRP, B2M, and NGAL.

The biomarker combination may further comprise a biomarker combination selected from the group consisting of:
(i) anti-ECPKA antibody, anti-TK1 antibody, anti-granulin antibody, anti-Her-2 antibody, anti-hP53 antibody, anti-c-myc antibody, cP53, hPKA, cTK1, AFP, CEA, PSA, CRP, and B2M;
(ii) anti-ECPKA antibody, anti-TK1 antibody, anti-granulin antibody, anti-Her-2 antibody, anti-hP53 antibody, anti-c-myc antibody, cTK1, AFP, and CRP;
(iii) anti-granulin antibody, anti-Her-2 antibody, anti-hP53 antibody, anti-c-myc antibody, cP53, hPKA, cTK1, AFP, CEA, PSA, CRP, B2M, and NGAL;
(iv) anti-ECPKA antibody, anti-TK1 antibody, anti-granulin antibody, anti-Her-2 antibody, anti-hP53 antibody, anti-c-myc antibody, cTK1, AFP, CEA, PSA, and CRP;
(v) anti-ECPKA antibody, anti-TK1 antibody, anti-granulin antibody, anti-Her-2 antibody, anti-hP53 antibody, anti-c-myc antibody, cTK1, AFP, CEA, PSA, CRP, and B2M; and
(vi) anti-ECPKA antibody, anti-TK1 antibody, anti-granulin antibody, anti-Her-2 antibody, anti-hP53 antibody, anti-c-myc antibody, cTK1, AFP, and CRP.

Also disclosed but not part of the invention, there is provided a composition for diagnosing lung cancer, comprising:
an agent for measuring the expression level of a gene or the expression level of a protein or a fragment thereof for a combination of anti-ECPKA antibody, anti-TK1 antibody, anti-granulin antibody, and anti-Her-2 antibody; and one or more biomarkers selected from the group consisting of anti-hP53 antibody, anti-c-myc antibody, and AFP.

The biomarker combination may further comprise one or more biomarkers selected from the group consisting of cP53 and cTK1.

The biomarker combination may comprise a biomarker combination selected from the group consisting of:
(i) anti-ECPKA antibody, anti-TK1 antibody, anti-granulin antibody, anti-Her-2 antibody, anti-hP53 antibody, and anti-c-myc antibody;
(ii) anti-ECPKA antibody, anti-TK1 antibody, anti-granulin antibody, anti-Her-2 antibody, anti-hP53 antibody, anti-c-myc antibody, and cP53;
(iii) anti-ECPKA antibody, anti-TK1 antibody, anti-granulin antibody, anti-Her-2 antibody, anti-hP53 antibody, anti-c-myc antibody, and cTK1; and
(iv) anti-ECPKA antibody, anti-TK1 antibody, anti-granulin antibody, anti-Her-2 antibody, anti-hP53 antibody, anti-c-myc antibody, and AFP.

The biomarker combination may further comprise a biomarker combination selected from the group consisting of:
(i) anti-ECPKA antibody, anti-TK1 antibody, anti-granulin antibody, anti-Her-2 antibody, anti-hP53 antibody, anti-c-myc antibody, cP53, hPKA, cTK1, AFP, CEA, PSA, CRP, and B2M;
(ii) anti-ECPKA antibody, anti-TK1 antibody, anti-granulin antibody, anti-Her-2 antibody, anti-hP53 antibody, anti-c-myc antibody, cP53, hPKA, cTK1, AFP, CEA, PSA, CRP, B2M, and NGAL;
(iii) anti-ECPKA antibody, anti-TK1 antibody, anti-granulin antibody, anti-Her-2 antibody, anti-hP53 antibody, anti-c-myc antibody, cTK1, AFP, CEA, CRP, B2M, and NGAL;
(iv) anti-ECPKA antibody, anti-TK1 antibody, anti-granulin antibody, anti-Her-2 antibody, anti-hP53 antibody, anti-c-myc antibody, cTK1, AFP, CEA, PSA, and CRP;
(v) anti-ECPKA antibody, anti-TK1 antibody, anti-granulin antibody, anti-Her-2 antibody, anti-hP53 antibody, anti-c-myc antibody, cTK1, AFP, CEA, PSA, CRP, and B2M; and
(vi) anti-ECPKA antibody, anti-TK1 antibody, anti-granulin antibody, anti-Her-2 antibody, anti-hP53 antibody, anti-c-myc antibody, cTK1, AFP, CEA, PSA, CRP, B2M, and NGAL.

The biomarker combination may further comprise a biomarker combination selected from the group consisting of:
(i) anti-ECPKA antibody, anti-TK1 antibody, anti-granulin antibody, anti-Her-2 antibody, anti-hP53 antibody, anti-c-myc antibody, cP53, and hPKA;
(ii) anti-ECPKA antibody, anti-TK1 antibody, anti-granulin antibody, anti-Her-2 antibody, anti-hP53 antibody, anti-c-myc antibody, cP53, hPKA, and cTK1;
(iii) anti-ECPKA antibody, anti-TK1 antibody, anti-granulin antibody, anti-Her-2 antibody, anti-hP53 antibody, anti-c-myc antibody, cP53, hPKA, cTK1, AFP, CEA, PSA, and CRP;
(iv) anti-ECPKA antibody, anti-TK1 antibody, anti-granulin antibody, anti-Her-2 antibody, anti-hP53 antibody, anti-c-myc antibody, cTK1, AFP, and CRP; and
(v) anti-ECPKA antibody, anti-TK1 antibody, anti-granulin antibody, anti-Her-2 antibody, anti-hP53 antibody, anti-c-myc antibody, AFP, CRP, and B2M.

Also disclosed but not part of the invention, there is provided a composition for diagnosing ovarian cancer, comprising:
an agent for measuring the expression level of a gene or the expression level of a protein or a fragment thereof for a combination of anti-ECPKA antibody, anti-TK1 antibody, anti-granulin antibody, and anti-Her-2 antibody; and one or more biomarkers selected from the group consisting of anti-hP53 antibody, anti-c-myc antibody, AFP, CEA, CRP, and B2M.

The biomarker combination may further comprise one or more biomarkers selected from the group consisting of cP53, hPKA, cTK1, PSA, and NGAL.

The biomarker combination may comprise a biomarker combination selected from the group consisting of:
(i) anti-ECPKA antibody, anti-TK1 antibody, anti-granulin antibody, anti-Her-2 antibody, anti-hP53 antibody, anti-c-myc antibody, cP53, hPKA, cTK1, AFP, CEA, PSA, CRP, and B2M; and
(ii) anti-ECPKA antibody, anti-TK1 antibody, anti-granulin antibody, anti-Her-2 antibody, anti-hP53 antibody, anti-c-myc antibody, cP53, hPKA, cTK1, AFP, CEA, PSA, CRP, B2M, and NGAL.

The biomarker combination may further comprise a biomarker combination selected from the group consisting of:
(i) anti-ECPKA antibody, anti-TK1 antibody, anti-granulin antibody, anti-Her-2 antibody, anti-hP53 antibody, anti-c-myc antibody, cTK1, AFP, CEA, PSA, CRP, and B2M; and
(ii) anti-ECPKA antibody, anti-TK1 antibody, anti-granulin antibody, anti-Her-2 antibody, anti-hP53 antibody, anti-c-myc antibody, AFP, CEA, CRP, and B2M.

The biomarker combination may further comprise a biomarker combination selected from the group consisting of:
(i) anti-ECPKA antibody, anti-TK1 antibody, anti-granulin antibody, anti-Her-2 antibody, anti-hP53 antibody, anti-c-myc antibody, cTK1, AFP, CEA, CRP, B2M, and NGAL;
(ii) anti-ECPKA antibody, anti-TK1 antibody, anti-granulin antibody, anti-Her-2 antibody, anti-hP53 antibody, anti-c-myc antibody, cTK1, AFP, CEA, PSA, CRP, B2M, and NGAL; and
(iii) anti-ECPKA antibody, anti-TK1 antibody, anti-granulin antibody, anti-Her-2 antibody, anti-hP53 antibody, anti-c-myc antibody, AFP, TK1, PSA, CEA, CRP, and B2M.

Also disclosed but not part of the invention, there is provided a composition for diagnosing pancreatic cancer, comprising:
an agent for measuring the expression level of a gene or the expression level of a protein or a fragment thereof for a combination of anti-ECPKA antibody, anti-TK1 antibody, anti-granulin antibody, and anti-Her-2 antibody; and one or more biomarkers selected from the group consisting of anti-hP53 antibody, anti-c-myc antibody, cTK1, AFP, and CRP.

The biomarker combination may further comprise one or more biomarkers selected from the group consisting of CEA, PSA, B2M, and NGAL.

The biomarker combination may comprise a biomarker combination selected from the group consisting of:
(i) anti-ECPKA antibody, anti-TK1 antibody, anti-granulin antibody, anti-Her-2 antibody, anti-hP53 antibody, anti-c-myc antibody, cTK1, AFP, CEA, CRP, B2M, and NGAL; and
(ii) anti-ECPKA antibody, anti-TK1 antibody, anti-granulin antibody, anti-Her-2 antibody, anti-hP53 antibody, anti-c-myc antibody, cTK1, AFP, CEA, PSA, CRP, B2M, and NGAL.

The biomarker combination may further comprise a biomarker combination selected from the group consisting of:
(i) anti-ECPKA antibody, anti-TK1 antibody, anti-granulin antibody, anti-Her-2 antibody, anti-hP53 antibody, anti-c-myc antibody, cP53, hPKA, cTK1, and AFP;
(ii) anti-ECPKA antibody, anti-TK1 antibody, anti-granulin antibody, anti-Her-2 antibody, anti-hP53 antibody, anti-c-myc antibody, and cTK1; and
(iii) anti-ECPKA antibody, anti-TK1 antibody, anti-granulin antibody, anti-Her-2 antibody, anti-hP53 antibody, anti-c-myc antibody, cTK1, and AFP.

The biomarker combination may further comprise a biomarker combination selected from the group consisting of:
(i) anti-ECPKA antibody, anti-TK1 antibody, anti-granulin antibody, anti-Her-2 antibody, anti-hP53 antibody, and anti-c-myc antibody;
(ii) anti-ECPKA antibody, anti-TK1 antibody, anti-granulin antibody, anti-Her-2 antibody, anti-hP53 antibody, anti-c-myc antibody, cP53, hPKA, and cTK1;
(iii) anti-ECPKA antibody, anti-TK1 antibody, anti-granulin antibody, anti-Her-2 antibody, anti-hP53 antibody, anti-c-myc antibody, cP53, hPKA, cTK1, AFP, CEA, PSA, CRP, and B2M;
(iv) anti-ECPKA antibody, anti-TK1 antibody, anti-granulin antibody, anti-Her-2 antibody, anti-hP53 antibody, anti-c-myc antibody, cP53, hPKA, cTK1, AFP, CEA, PSA, CRP, B2M, and NGAL;
(v) anti-ECPKA antibody, anti-TK1 antibody, anti-granulin antibody, anti-Her-2 antibody, anti-hP53 antibody, anti-c-myc antibody, cTK1, AFP, and CRP;
(vi) anti-ECPKA antibody, anti-TK1 antibody, anti-granulin antibody, anti-Her-2 antibody, anti-hP53 antibody, and anti-c-myc antibody;
(vii) anti-ECPKA antibody, anti-TK1 antibody, anti-granulin antibody, anti-Her-2 antibody, anti-hP53 antibody, anti-c-myc antibody, cTK1, AFP, CEA, PSA, CRP, and B2M; and
(viii) anti-ECPKA antibody, anti-TK1 antibody, anti-granulin antibody, anti-Her-2 antibody, TK1, AFP, and CRP.

Also disclosed but not part of the invention, there is provided a composition for diagnosing prostate cancer, comprising:
an agent for measuring the expression level of a gene or the expression level of a protein or a fragment thereof for a combination of anti-ECPKA antibody, anti-TK1 antibody, anti-granulin antibody, and anti-Her-2 antibody; and one or more biomarkers selected from the group consisting of anti-hP53 antibody, anti-c-myc antibody, AFP, and PSA.

The biomarker combination may further comprise one or more biomarkers selected from the group consisting of cTK1, CEA, CRP, B2M, and NGAL.

The biomarker combination may comprise a biomarker combination selected from the group consisting of:
(i) anti-ECPKA antibody, anti-TK1 antibody, anti-granulin antibody, anti-Her-2 antibody, anti-hP53 antibody, anti-c-myc antibody, cTK1, AFP, CEA, CRP, B2M, and NGAL; and
(ii) anti-ECPKA antibody, anti-TK1 antibody, anti-granulin antibody, anti-Her-2 antibody, anti-hP53 antibody, anti-c-myc antibody, cTK1, AFP, CEA, PSA, CRP, B2M, and NGAL.

The biomarker combination may further comprise a biomarker combination selected from the group consisting of:
(i) anti-ECPKA antibody, anti-TK1 antibody, anti-granulin antibody, anti-Her-2 antibody, anti-hP53 antibody, anti-c-myc antibody, cP53, and hPKA;
(ii) anti-ECPKA antibody, anti-TK1 antibody, anti-granulin antibody, anti-Her-2 antibody, anti-hP53 antibody, anti-c-myc antibody, cP53, hPKA, and cTK1;
(iii) anti-ECPKA antibody, anti-TK1 antibody, anti-granulin antibody, anti-Her-2 antibody, anti-hP53 antibody, anti-c-myc antibody, cP53, hPKA, cTK1, AFP, CEA, PSA, CRP, and B2M;
(iv) anti-ECPKA antibody, anti-TK1 antibody, anti-granulin antibody, anti-Her-2 antibody, anti-hP53 antibody, anti-c-myc antibody, cP53, hPKA, cTK1, AFP, CEA, PSA, CRP, B2M, and NGAL; and
(v) anti-ECPKA antibody, anti-TK1 antibody, anti-granulin antibody, anti-Her-2 antibody, anti-hP53 antibody, anti-c-myc antibody, cTK1, AFP, CEA, PSA, CRP, and B2M.

The biomarker combination may further comprise a biomarker combination selected from the group consisting of:
(i) anti-ECPKA antibody, anti-TK1 antibody, anti-granulin antibody, anti-Her-2 antibody, anti-hP53 antibody, anti-c-myc antibody, and cP53;
(ii) anti-ECPKA antibody, anti-TK1 antibody, anti-granulin antibody, anti-Her-2 antibody, anti-hP53 antibody, anti-c-myc antibody, cP53, hPKA, cTK1, and AFP;
(iii) anti-ECPKA antibody, anti-TK1 antibody, anti-granulin antibody, anti-Her-2 antibody, anti-hP53 antibody, anti-c-myc antibody, cP53, hPKA, cTK1, AFP, CEA, and PSA;
(iv) anti-ECPKA antibody, anti-TK1 antibody, anti-granulin antibody, anti-Her-2 antibody, anti-hP53 antibody, anti-c-myc antibody, cTK1, AFP, and CRP;
(v) anti-ECPKA antibody, anti-TK1 antibody, anti-granulin antibody, anti-Her-2 antibody, anti-hP53 antibody, anti-c-myc antibody, and cTK1;
(vi) anti-ECPKA antibody, anti-TK1 antibody, anti-granulin antibody, anti-Her-2 antibody, anti-hP53 antibody, anti-c-myc antibody, cTK1, and AFP;
(vii) anti-ECPKA antibody, anti-TK1 antibody, anti-granulin antibody, anti-Her-2 antibody, anti-hP53 antibody, anti-c-myc antibody, cTK1, AFP, and CEA;
(viii) anti-ECPKA antibody, anti-TK1 antibody, anti-granulin antibody, anti-Her-2 antibody, anti-hP53 antibody, anti-c-myc antibody, cTK1, AFP, CEA, and PSA;
(ix) anti-ECPKA antibody, anti-TK1 antibody, anti-granulin antibody, anti-Her-2 antibody, anti-hP53 antibody, anti-c-myc antibody, cTK1, AFP, and CRP;
(x) anti-granulin antibody, anti-Her-2 antibody, anti-hP53 antibody, anti-c-myc antibody, cTK1, AFP, CEA, PSA, CRP, B2M, and NGAL; and
(xi) anti-ECPKA antibody, anti-TK1 antibody, anti-granulin antibody, anti-Her-2 antibody, anti-hP53 antibody, anti-c-myc antibody, AFP, and PSA.

Also disclosed but not part of the invention, there is provided a composition for diagnosing renal cancer, comprising:
an agent for measuring the expression level of a gene or the expression level of a protein or a fragment thereof for a combination of anti-ECPKA antibody, anti-TK1 antibody, anti-granulin antibody, and anti-Her-2 antibody; and one or more biomarkers selected from the group consisting of anti-hP53 antibody, anti-c-myc antibody, AFP, CEA, CRP, and B2M.

The biomarker combination may further comprise one or more biomarkers selected from the group consisting of cTK1, PSA, and NGAL.

The biomarker combination may comprise a biomarker combination selected from the group consisting of:
(i) anti-ECPKA antibody, anti-TK1 antibody, anti-granulin antibody, anti-Her-2 antibody, anti-hP53 antibody, anti-c-myc antibody, cTK1, AFP, CEA, CRP, B2M, and NGAL; and
(ii) anti-ECPKA antibody, anti-TK1 antibody, anti-granulin antibody, anti-Her-2 antibody, anti-hP53 antibody, anti-c-myc antibody, cTK1, AFP, CEA, PSA, CRP, B2M, and NGAL.

The biomarker combination may further comprise a biomarker combination selected from the group consisting of:
(i) anti-ECPKA antibody, anti-TK1 antibody, anti-granulin antibody, anti-Her-2 antibody, anti-hP53 antibody, anti-c-myc antibody, cP53, hPKA, cTK1, AFP, CEA, PSA, CRP, and B2M; and
(ii) anti-ECPKA antibody, anti-TK1 antibody, anti-granulin antibody, anti-Her-2 antibody, anti-hP53 antibody, anti-c-myc antibody, cTK1, AFP, CEA, PSA, CRP, and B2M.

The biomarker combination may further comprise a biomarker combination selected from the group consisting of:
(i) anti-ECPKA antibody, anti-TK1 antibody, anti-granulin antibody, anti-Her-2 antibody, anti-hP53 antibody, anti-c-myc antibody, AFP, CEA, CRP, and B2M; and
(ii) anti-ECPKA antibody, anti-TK1 antibody, anti-granulin antibody, anti-Her-2 antibody, anti-hP53 antibody, anti-c-myc antibody, AFP, CEA, CRP, B2M, and NGAL.

In the anti-ECPKA antibody, the term "ECPKA" refers to extracellular protein kinase A. The protein kinase A (PKA) is also referred to as cAMP-dependent protein kinase. For humans, the ECPKA may comprise an amino acid sequence represented by Uniprot No. P17612. For dogs, the ECPKA may comprise an amino acid sequence represented by Uniprot No. Q8MJ44. The anti-ECPKA antibody may be an antibody specific for ECPKA or an antigen binding fragment thereof.

In the anti-TK1 antibody or TK1, the term "TK1" refers to thymidine kinase 1 (TK1). The TK1 may be cytosolic thymidine kinase 1 (cTK1). For humans, the TK1 may comprise an amino acid sequence represented by Uniprot No. P04183. For dogs, the TK1 may comprise an amino acid sequence represented by Uniprot No. E2QXP4. The anti-TK1 antibody may be an antibody specific for TK1 or an antigen binding fragment thereof.

In the anti-granulin antibody, the term "granulin" may also be referred to as GRN, CLN11, GEP, GP88, PCDGF, PEPI, PGranulin, or granulin precursor. The granulin may be in a cleaved form from its precursor, progranulin. The granulin may function in development, inflammation, cell proliferation, and protein homeostasis. For humans, the granulin may comprise an amino acid sequence represented by Uniprot No. P28799. For dogs, the granulin may comprise an amino acid sequence represented by Uniprot No. E2RHN1. The anti-granulin antibody may be an antibody specific for granulin or progranulin or an antigen binding fragment thereof.

In the anti-Her-2 antibody, the term "Her-2" refers to human epidermal growth factor receptor 2. The Her-2 may also be referred to as ERBB2, CD340, HER-2/neu, HER2, MLN 19, NEU, NGL, TKR1, or erb-b2 receptor tyrosine kinase 2. The Her-2 may be a member of the ErbB family, a growth factor receptor that promotes cell growth and division. The Her-2 may be overexpressed in breast cancer or gastric cancer. For humans, the Her-2 may comprise an amino acid sequence represented by Uniprot No. P04626. For dogs, the Her-2 may comprise an amino acid sequence represented by Uniprot No. Q18735. The anti-Her-2 antibody may be an antibody specific for Her-2 or an antigen binding fragment thereof.

In the anti-hP53 antibody, the term "hP53" refers to human P53. The P53 may also be referred to as TP53, BCC7, LFS1, P53, TRP53, tumor protein p53, or BMFS5. The P53 may be involved in the development of most cancers in the human body, including gastric cancer, colorectal cancer, prostate cancer, and lung cancer. The normal p53 gene has the function of inducing cell generation and apoptosis, but the p53 gene mutation may cause cancer by losing the ability to control cell growth and apoptosis. For humans, the P53 may comprise an amino acid sequence represented by Uniprot No. P04637. For dogs, the P53 may comprise an amino acid sequence represented by Uniprot No. Q29537 or E7FIY6. The anti-hP53 antibody may be an antibody specific for human P53 or an antigen binding fragment thereof.

In the anti-c-myc antibody, the term "c-myc" is also referred to as MYC, MRTL, MYCC, bHLHe39, c-Myc, v-myc avian myelocytomatosis viral oncogene homolog, MYC proto-oncogene, bHLH transcription factor, or myc. The c-myc may be a proto-oncogene encoding a transcription factor. The c-myc not only express factors that regulate cell cycle regulation and division, but may also play a role in function in cancer cell immune evasion mechanisms and cancer cell metabolic signals. For humans, the c-myc may comprise an amino acid sequence represented by Uniprot No. P01106. For dogs, the c-myc may comprise an amino acid sequence represented by Uniprot No. Q28350. The anti-c-myc antibody may be an antibody specific for c-myc or an antigen binding fragment thereof.

The AFP refers to alpha-fetoprotein. The AFP may also be referred to as AFPD, FETA, or HPalpha fetoprotein. The AFP may be a plasma protein found in a human fetus. The AFP is produced in the endoderm of the yolk egg in the early stages of development, but is produced in the liver in the later stage of development, and may decrease at birth, so that the concentration in the serum of adults is 10 ng/mL or less, and may increase again when a pathological condition occurs. For humans, the AFP may comprise an amino acid sequence represented by Uniprot No. P02771. For dogs, the AFP may comprise an amino acid sequence represented by Uniprot No. Q8MJU5.

The CEA refers to carcinoembryonic antigen. The CEA may be a glycoprotein involved in cell adhesion. The CEA has a low positive predictive value and is not often used as a screening test, but may be used to determine the stage, prognosis, and recurrence of colorectal cancer. For humans, the CEA may comprise an amino acid sequence represented by Uniprot No. P06731. For dogs, the CEA may comprise an amino acid sequence represented by Uniprot No. E2RPZ7.

The CRP refers to C-reactive protein. The CRP may be a ring-shaped pentameric protein present in plasma. The CRP may also be referred to as PTX1 (pentraxin-related). For humans, the CRP may comprise an amino acid sequence represented by Uniprot No. P02741. For dogs, the CRP may comprise an amino acid sequence represented by Uniprot No. E2R5J0.

The B2M refers to beta-2 microglobulin (β2 microglobulin). The B2M may be a component of MHC class I molecules. The B2M may also be referred to as IMD43. For humans, the B2M may comprise an amino acid sequence represented by Uniprot No. P61769. For dogs, the B2M may comprise an amino acid sequence represented by Uniprot No. A0A5F4C4Q1.

The NGAL refers to neutrophil gelatinase-associated lipocalin. The NGAL may be involved in innate immunity that sequesters iron and prevents its use by bacteria. The NGAL may also be referred to as LCN2, 24p3, MSFI, p25, or lipocalin 2. For humans, the NGAL may comprise an amino acid sequence represented by Uniprot No. P80188. For dogs, the NGAL may comprise an amino acid sequence represented by Uniprot No. E2RSM4.

The PSA refers to prostate-specific antigen. The PSA may be a protein present in male plasma. The PSA may also be referred to as gamma-seminoprotein, kallikrein-3, KLK3, APS, KLK2A1, PSA, hK3, or kallikrein-related peptidase 3. The PSA is secreted from the epithelial cells of the prostate, and may help sperm be ejaculated, and may serve to induce sperm into the uterus by dissolving vaginal mucus. The PSA is present in small amounts in healthy people, but its expression may increase in cases of prostate cancer or other prostate diseases. However, The PSA is not the only indicator of prostate cancer and may also diagnose prostatitis or benign prostatic hyperplasia. For humans, the PSA may comprise an amino acid sequence represented by Uniprot No. P07288. For dogs, the PSA may comprise an amino acid sequence represented by Uniprot No. P0728.

The cP53 refers to canine P53. The P53 is the same as described above.

The hPKA refers to human PKA. The PKA is the same as described above.

The fragment is a part of the protein and may be an immunogenic polypeptide.

The term "gene" is a unit of genetic information and refers to a nucleic acid sequence that encodes a substance having a function. The expression level of the gene may be the expression level of mRNA that encodes the protein. The expression level of mRNA may be a relative amount or an absolute amount of mRNA. Measuring the expression level of the gene may mean measuring the amount of mRNA.

The expression level of the protein or fragment thereof may be a relative amount or an absolute amount of the protein or fragment thereof. Measuring the expression level of the protein or fragment thereof may mean measuring the amount of the protein or fragment thereof.

The expression level may be increased compared to the expression level of a normal control group.

The agent may be an antibody or an antigen binding fragment thereof, or an aptamer that specifically binds to the protein or fragment thereof. The antibody may be a polyclonal antibody or a monoclonal antibody. The term "antibody" may be used interchangeably with the term "immunoglobulin." The antibody may be a polyclonal antibody or a monoclonal antibody. The antibody may be a full length antibody. The antigen binding fragment refers to a polypeptide comprising an antigen binding site. The antigen binding fragment may be a single-domain antibody, Fab, Fab', or scFv. The antibody or antigen binding fragment may be attached to a solid support. The solid support is, for example, the surface of a metal chip, plate, or well. The antibody or antigen binding fragment may be an antibody or an antigen binding fragment. The aptamer may be a single-stranded nucleic acid (DNA, RNA, or modified nucleic acid) or a peptide that specifically binds to the protein or fragment thereof.

The agent may be a nucleic acid comprising a polynucleotide identical to or complementary to a polynucleotide that encodes the protein or fragment thereof. The nucleic acid may be a primer, a probe, or an antisense oligonucleotide. The primer, probe, or antisense oligonucleotide may be labeled at its end or inside with a fluorescent substance, a chemiluminescent substance, or a radioactive isotope, etc.

The term "diagnosis" refers to determining the name of the disease, and may include the name of the cancer, the condition of the disease, the stage, the etiology, the presence or absence of complications, prognosis, and recurrence, etc.

In another aspect, there is provided a kit for diagnosing gastric cancer as defined in claim 9.

Also disclosed but not part of the invention, there is provided a kit for diagnosing bladder cancer, comprising:
an agent for measuring the expression level of a gene or the expression level of a protein or a fragment thereof for a combination of anti-ECPKA antibody, anti-TK1 antibody, anti-granulin antibody, and anti-Her-2 antibody; and one or more biomarkers selected from the group consisting of cTK1, CEA, AFP, and CRP.

Also disclosed but not part of the invention, there is provided a kit for diagnosing breast cancer, comprising:
an agent for measuring the expression level of a gene or the expression level of a protein or a fragment thereof for a combination of anti-ECPKA antibody, anti-TK1 antibody, anti-granulin antibody, and anti-Her-2 antibody; and one or more biomarkers selected from the group consisting of cTK1, AFP, B2M, and NGAL.

Also disclosed but not part of the invention, there is provided a kit for diagnosing cervical cancer, comprising:
an agent for measuring the expression level of a gene or the expression level of a protein or a fragment thereof for a combination of anti-ECPKA antibody, anti-TK1 antibody, anti-granulin antibody, and anti-Her-2 antibody; and one or more biomarkers selected from the group consisting of AFP and B2M.

Also disclosed but not part of the invention, there is provided a kit for diagnosing colorectal cancer: comprising
an agent for measuring the expression level of a gene or the expression level of a protein or a fragment thereof for a combination of anti-ECPKA antibody, anti-TK1 antibody, anti-granulin antibody, and anti-Her-2 antibody; and one or more biomarkers selected from the group consisting of AFP, CEA, CRP, and NGAL.

Also disclosed but not part of the invention, there is provided a kit for diagnosing liver cancer, comprising:
an agent for measuring the expression level of a gene or the expression level of a protein or a fragment thereof for a combination of anti-ECPKA antibody, anti-TK1 antibody, anti-granulin antibody, and anti-Her-2 antibody; and one or more biomarkers selected from the group consisting of anti-hP53 antibody, anti-c-myc antibody, AFP, and cTK1.

Also disclosed but not part of the invention, there is provided a kit for diagnosing lung cancer, comprising:
an agent for measuring the expression level of a gene or the expression level of a protein or a fragment thereof for a combination of anti-ECPKA antibody, anti-TK1 antibody, anti-granulin antibody, and anti-Her-2 antibody; and one or more biomarkers selected from the group consisting of anti-hP53 antibody, anti-c-myc antibody, and AFP.

Also disclosed but not part of the invention, there is provided a kit for diagnosing ovarian cancer, comprising:
an agent for measuring the expression level of a gene or the expression level of a protein or a fragment thereof for a combination of anti-ECPKA antibody, anti-TK1 antibody, anti-granulin antibody, and anti-Her-2 antibody; and one or more biomarkers selected from the group consisting of anti-hP53 antibody, anti-c-myc antibody, AFP, CEA, CRP, and B2M.

Also disclosed but not part of the invention, there is provided a kit for diagnosing pancreatic cancer, comprising:
an agent for measuring the expression level of a gene or the expression level of a protein or a fragment thereof for a combination of anti-ECPKA antibody, anti-TK1 antibody, anti-granulin antibody, and anti-Her-2 antibody; and one or more biomarkers selected from the group consisting of anti-hP53 antibody, anti-c-myc antibody, cTK1, AFP, and CRP.

Also disclosed but not part of the invention, there is provided a kit for diagnosing prostate cancer, comprising:
an agent for measuring the expression level of a gene or the expression level of a protein or a fragment thereof for a combination of anti-ECPKA antibody, anti-TK1 antibody, anti-granulin antibody, and anti-Her-2 antibody; and one or more biomarkers selected from the group consisting of anti-hP53 antibody, anti-c-myc antibody, AFP, and PSA.

Also disclosed but not part of the invention, there is provided a kit for diagnosing renal cancer, comprising:
an agent for measuring the expression level of a gene or the expression level of a protein or a fragment thereof for a combination of anti-ECPKA antibody, anti-TK1 antibody, anti-granulin antibody, and anti-Her-2 antibody; and one or more biomarkers selected from the group consisting of anti-hP53 antibody, anti-c-myc antibody, AFP, CEA, CRP, and B2M.

The kit may further comprise a sample necessary for cancer diagnosis. The kit may comprise a solid support, a substrate for immunological detection of an antibody or an antigen binding fragment, a suitable buffer solution, a chromogenic enzyme, a secondary antibody labeled with a fluorescent substance, or a chromogenic substrate. The kit may comprise polymerase, a buffering agent, nucleic acid, coenzyme, a fluorescent substance, or a combination thereof for detection of nucleic acid. The polymerase is, for example, Taq polymerase.

The kit may be a kit capable of digital quantification of signals.

In another aspect, there is provided a method for detecting a biomarker for diagnosing cancer, as defined in claim 11.

Also disclosed but not part of the invention, there is provided a method for detecting a biomarker for diagnosing bladder cancer, comprising the steps of:
measuring the expression level of a gene or the expression level of a protein or a fragment thereof for a combination of anti-ECPKA antibody, anti-TK1 antibody, anti-granulin antibody, and anti-Her-2 antibody; and one or more biomarkers selected from the group consisting of cTK1, CEA, AFP, and CRP in a biological sample isolated from a subject suspected of having bladder cancer; and
comparing the measured expression level with the expression level of a gene or the expression level of a protein or a fragment thereof in a normal control group.

Also disclosed but not part of the invention, there is provided a method for detecting a biomarker for diagnosing breast cancer, comprising the steps of:
measuring the expression level of a gene or the expression level of a protein or a fragment thereof for a combination of anti-ECPKA antibody, anti-TK1 antibody, anti-granulin antibody, and anti-Her-2 antibody; and one or more biomarkers selected from the group consisting of cTK1, AFP, B2M, and NGAL in a biological sample isolated from a subject suspected of having breast cancer; and
comparing the measured expression level with the expression level of a gene or the expression level of a protein or a fragment thereof in a normal control group.

Also disclosed but not part of the invention, there is provided a method for detecting a biomarker for diagnosing cervical cancer, comprising the steps of:
measuring the expression level of a gene or the expression level of a protein or a fragment thereof for a combination of anti-ECPKA antibody, anti-TK1 antibody, anti-granulin antibody, and anti-Her-2 antibody; and one or more biomarkers selected from the group consisting of AFP and B2M in a biological sample isolated from a subject suspected of having cervical cancer; and
measuring the expression level of a gene or the expression level of a protein or a fragment thereof selected from the group consisting of anti-ECPKA antibody, anti-TK1 antibody, anti-granulin antibody, anti-Her-2 antibody, AFP, and B2M; and
comparing the measured expression level with the expression level of a gene or the expression level of a protein or a fragment thereof in a normal control group.

Also disclosed but not part of the invention, there is provided a method for detecting a biomarker for diagnosing colorectal cancer, comprising the steps of:
measuring the expression level of a gene or the expression level of a protein or a fragment thereof for a combination of anti-ECPKA antibody, anti-TK1 antibody, anti-granulin antibody, and anti-Her-2 antibody; and one or more biomarkers selected from the group consisting of AFP, CEA, CRP, and NGAL in a biological sample isolated from a subject suspected of having colorectal cancer; and
comparing the measured expression level with the expression level of a gene or the expression level of a protein or a fragment thereof in a normal control group.

Also disclosed but not part of the invention, there is provided a method for detecting a biomarker for diagnosing liver cancer, comprising the steps of:
measuring the expression level of a gene or the expression level of a protein or a fragment thereof for a combination of anti-ECPKA antibody, anti-TK1 antibody, anti-granulin antibody, and anti-Her-2 antibody; and one or more biomarkers selected from the group consisting of anti-hP53 antibody, anti-c-myc antibody, AFP, and cTK1 in a biological sample isolated from a subject suspected of having liver cancer; and
comparing the measured expression level with the expression level of a gene or the expression level of a protein or a fragment thereof in a normal control group.

Also disclosed but not part of the invention, there is provided a method for detecting a biomarker for diagnosing lung cancer, comprising the steps of:
measuring the expression level of a gene or the expression level of a protein or a fragment thereof for a combination of anti-ECPKA antibody, anti-TK1 antibody, anti-granulin antibody, and anti-Her-2 antibody; and one or more biomarkers selected from the group consisting of anti-hP53 antibody, anti-c-myc antibody, and AFP in a biological sample isolated from a subject suspected of having lung cancer; and
comparing the measured expression level with the expression level of a gene or the expression level of a protein or a fragment thereof in a normal control group.

Also disclosed but not part of the invention, there is provided a method for detecting a biomarker for diagnosing ovarian cancer, comprising the steps of:
measuring the expression level of a gene or the expression level of a protein or a fragment thereof for a combination of anti-ECPKA antibody, anti-TK1 antibody, anti-granulin antibody, and anti-Her-2 antibody; and one or more biomarkers selected from the group consisting of anti-hP53 antibody, anti-c-myc antibody, AFP, CEA, CRP, and B2M in a biological sample isolated from a subject suspected of having ovarian cancer; and
comparing the measured expression level with the expression level of a gene or the expression level of a protein or a fragment thereof in a normal control group.

Also disclosed but not part of the invention, there is provided a method for detecting a biomarker for diagnosing pancreatic cancer, comprising the steps of:
measuring the expression level of a gene or the expression level of a protein or a fragment thereof for a combination of anti-ECPKA antibody, anti-TK1 antibody, anti-granulin antibody, and anti-Her-2 antibody; and one or more biomarkers selected from the group consisting of anti-hP53 antibody, anti-c-myc antibody, cTK1, AFP, and CRP in a biological sample isolated from a subject suspected of having pancreatic cancer; and
comparing the measured expression level with the expression level of a gene or the expression level of a protein or a fragment thereof in a normal control group.

Also disclosed but not part of the invention, there is provided a method for detecting a biomarker for diagnosing prostate cancer, comprising the steps of:
measuring the expression level of a gene or the expression level of a protein or a fragment thereof for a combination of anti-ECPKA antibody, anti-TK1 antibody, anti-granulin antibody, and anti-Her-2 antibody; and one or more biomarkers selected from the group consisting of anti-hP53 antibody, anti-c-myc antibody, AFP, and PSA in a biological sample isolated from a subject suspected of having prostate cancer; and
comparing the measured expression level with the expression level of a gene or the expression level of a protein or a fragment thereof in a normal control group.

Also disclosed but not part of the invention, there is provided a method for detecting a biomarker for diagnosing renal cancer, comprising the steps of:
measuring the expression level of a gene or the expression level of a protein or a fragment thereof for a combination of anti-ECPKA antibody, anti-TK1 antibody, anti-granulin antibody, and anti-Her-2 antibody; and one or more biomarkers selected from the group consisting of anti-hP53 antibody, anti-c-myc antibody, AFP, CEA, CRP, and B2M in a biological sample isolated from a subject suspected of having renal cancer; and
comparing the measured expression level with the expression level of a gene or the expression level of a protein or a fragment thereof in a normal control group.

The subject may be a mammal, such as a human, dog, cat, cow, horse, pig, sheep, goat, rabbit, mouse, hamster, hedgehog, ferret, or guinea pig. The subject may be a subject suffering from or suspected of having cancer, such as bladder cancer, breast cancer, cervical cancer, colorectal cancer, gastric cancer, liver cancer, lung cancer, ovarian cancer, pancreatic cancer, prostate cancer, and renal cancer.

The biological sample refers to a sample obtained from the subject. The biological sample may be blood, plasma, serum, bone marrow fluid, lymph fluid, saliva, tear fluid, mucosal fluid, amniotic fluid, or a combination thereof.

The measuring step may comprise the step of incubating the biological sample with an antibody or an antigen binding fragment thereof or an aptamer that specifically binds to the protein or fragment thereof, or incubating a polynucleotide identical to or complementary to a polynucleotide that encodes the gene.

The measuring step may be performed by electrophoresis, immunoblotting, enzyme-linked immunosorbent assay (ELISA), polymerase chain reaction, Northern blotting, polymerase amplification reaction (polymerase chain reaction: PCR), real-time polymerase chain reaction (real-time polymerase chain reaction or quantitative PCR: QPCR), reverse transcription polymerase chain reaction (rtPCR), protein chip, immunoprecipitation, microarray, electron microscopy, or a combination thereof. The electrophoresis may be SDS-PAGE, isoelectric electrophoresis, two-dimensional electrophoresis, or a combination thereof. The PCR may be real-time PCR or reverse transcription PCR.

The method comprises comparing the measured expression level with the expression level of a normal control group.

The expression level of the measured biomarker may be increased compared to the

## Claims

1. A composition for diagnosing gastric cancer, comprising:
agents for measuring the expression levels of (i) anti-ECPKA antibody, anti-TK1 antibody, anti-granulin antibody, and anti-Her-2 antibody; and (ii) one or more biomarkers selected from the group consisting of anti-hP53 antibody, anti-c-myc antibody, AFP, and cTK1,
wherein the agents are
an antibody or an antigen binding fragment thereof or an aptamer that specifically binds to the protein ; or
a nucleic acid comprising a polynucleotide identical to or complementary to a polynucleotide that encodes the protein,

2. The composition for diagnosing gastric cancer according to claim 1, wherein the biomarker combination further comprises one or more biomarkers selected from the group consisting of cP53, hPKA, CEA, PSA, CRP, B2M, and NGAL.

3. The composition for diagnosing gastric cancer according to claim 1, wherein the biomarker combination comprises a biomarker combination selected from the group consisting of:
(i) anti-ECPKA antibody, anti-TK1 antibody, anti-granulin antibody, anti-Her-2 antibody, anti-hP53 antibody, anti-c-myc antibody, cP53, hPKA, cTK1, AFP, CEA, PSA, CRP, and B2M; and
(ii) anti-ECPKA antibody, anti-TK1 antibody, anti-granulin antibody, anti-Her-2 antibody, anti-hP53 antibody, anti-c-myc antibody, cP53, hPKA, cTK1, AFP, CEA, PSA, CRP, B2M, and NGAL.

4. The composition for diagnosing gastric cancer according to claim 3, wherein the biomarker combination further comprises a biomarker combination selected from the group consisting of:
(i) anti-ECPKA antibody, anti-TK1 antibody, anti-granulin antibody, anti-Her-2 antibody, anti-hP53 antibody, anti-c-myc antibody, cP53, and hPKA; and
(ii) anti-ECPKA antibody, anti-TK1 antibody, anti-granulin antibody, anti-Her-2 antibody, anti-hP53 antibody, anti-c-myc antibody, cP53, hPKA, cTK1, and AFP.

5. The composition for diagnosing gastric cancer according to claim 3 or 4, wherein the biomarker combination further comprises a biomarker combination selected from the group consisting of:
(i) anti-ECPKA antibody, anti-TK1 antibody, anti-granulin antibody, anti-Her-2 antibody, anti-hP53 antibody, anti-c-myc antibody, cP53, hPKA, and cTK1;
(ii) anti-ECPKA antibody, anti-TK1 antibody, anti-granulin antibody, anti-Her-2 antibody, anti-hP53 antibody, anti-c-myc antibody, cP53, hPKA, cTK1, AFP, and CEA;
(iii) anti-ECPKA antibody, anti-TK1 antibody, anti-granulin antibody, anti-Her-2 antibody, anti-hP53 antibody, anti-c-myc antibody, cP53, hPKA, cTK1, AFP, CEA, and PSA;
(iv) anti-ECPKA antibody, anti-TK1 antibody, anti-granulin antibody, anti-Her-2 antibody, anti-hP53 antibody, anti-c-myc antibody, cP53, hPKA, cTK1, AFP, CEA, PSA, and CRP;
(v) anti-ECPKA antibody, anti-TK1 antibody, anti-granulin antibody, anti-Her-2 antibody, anti-hP53 antibody, anti-c-myc antibody, cTK1, AFP, CEA, CRP, B2M, and NGAL;
(vi) anti-ECPKA antibody, anti-TK1 antibody, anti-granulin antibody, anti-Her-2 antibody, anti-hP53 antibody, anti-c-myc antibody, cTK1, and AFP;
(vii) anti-ECPKA antibody, anti-TK1 antibody, anti-granulin antibody, anti-Her-2 antibody, anti-hP53 antibody, anti-c-myc antibody, cTK1, AFP, CEA, PSA, CRP, and B2M;
(viii) anti-ECPKA antibody, anti-TK1 antibody, anti-granulin antibody, anti-Her-2 antibody, anti-hP53 antibody, anti-c-myc antibody, cTK1, AFP, CEA, PSA, CRP, B2M, and NGAL;
(ix) anti-ECPKA antibody, anti-TK1 antibody, anti-granulin antibody, anti-Her-2 antibody, anti-hP53 antibody, anti-c-myc antibody, cTK1, AFP, CEA, CRP, B2M, and NGAL; and
(x) anti-ECPKA antibody, anti-TK1 antibody, anti-granulin antibody, anti-Her-2 antibody, anti-hP53 antibody, anti-c-myc antibody, AFP, and TK1.

6. The composition according to claim 1, wherein the expression level is increased compared to the expression level of a normal control group.

7. The composition according to claim 1, wherein the antibody is a polyclonal antibody or a monoclonal antibody.

8. The composition according to claim 1, wherein the nucleic acid is a primer, a probe, an antisense oligonucleotide, or an aptamer.

9. A kit for diagnosing gastric cancer, comprising:
first agents for measuring the expression levels of anti-ECPKA antibody, anti-TK1 antibody, anti-granulin antibody, and anti-Her-2 antibody; and
a second agent for measuring the expression levels of one or more biomarkers selected from the group consisting of anti-hP53 antibody, anti-c-myc antibody, AFP, and cTK1,
wherein the first agents or the second agent is
an antibody or an antigen binding fragment thereof or an aptamer that specifically binds to the protein ; or
a nucleic acid comprising a polynucleotide identical to or complementary to a polynucleotide that encodes the protein.

10. A method for detecting a biomarker for diagnosing gastric cancer, comprising the steps of:
measuring the expression levels of (i) anti-ECPKA antibody, anti-TK1 antibody, anti-granulin antibody, and anti-Her-2 antibody; and (ii) one or more biomarkers selected from the group consisting of anti-hP53 antibody, anti-c-myc antibody, AFP, and cTK1 in a biological sample isolated from a subject suspected of having gastric cancer; and
comparing the measured expression levels with the expression levels in a normal control group.

11. The method according to claim 10, wherein the biological sample is blood, plasma, serum, bone marrow fluid, lymph fluid, saliva, tear fluid, mucosal fluid, amniotic fluid, or a combination thereof.

12. The method according to claim 10, wherein the measuring step comprises the step of incubating the biological sample with an antibody or an antigen binding fragment thereof or an aptamer that specifically binds to the protein or fragment thereof, or incubating a polynucleotide identical to or complementary to a polynucleotide that encodes the gene.

13. The method according to claim 10, wherein the measuring step is performed by electrophoresis, immunoblotting, enzyme-linked immunosorbent assay (ELISA), polymerase chain reaction, Northern blotting, polymerase amplification reaction (polymerase chain reaction: PCR), protein chip, immunoprecipitation, microarray, electron microscopy, or a combination thereof.

## Patentansprüche

1. Zusammensetzung zum Diagnostizieren von Magenkrebs, umfassend:
Mittel zum Messen der Expressionsniveaus von (i) Anti-ECPKA-Antikörper, Anti-TK1-Antikörper, Anti-Granulin-Antikörper und Anti-Her-2-Antikörper; und (ii) einem oder mehreren Biomarkern, ausgewählt aus der Gruppe bestehend aus Anti-hP53-Antikörper, Anti-c-myc-Antikörper, AFP und cTK1,
wobei die Mittel Folgendes sind:
ein Antikörper oder ein Antigenbindungsfragment davon oder ein Aptamer sind, das spezifisch an das Protein bindet; oder
eine Nukleinsäure, die ein Polynukleotid umfasst, das identisch mit oder komplementär zu einem Polynukleotid ist, das das Protein kodiert.

2. Zusammensetzung zum Diagnostizieren von Magenkrebs nach Anspruch 1, wobei die Biomarkerkombination ferner einen oder mehrere Biomarker umfasst, ausgewählt aus der Gruppe bestehend aus cP53, hPKA, CEA, PSA, CRP, B2M und NGAL.

3. Zusammensetzung zum Diagnostizieren von Magenkrebs nach Anspruch 1, wobei die Biomarkerkombination eine Biomarkerkombination umfasst, die aus der Gruppe ausgewählt ist, die aus Folgendem besteht:
(i) Anti-ECPKA-Antikörper, Anti-TK1-Antikörper, Anti-Granulin-Antikörper, Anti-Her-2-Antikörper, Anti-hP53-Antikörper, Anti-c-myc-Antikörper, cP53, hPKA, cTK1, AFP, CEA, PSA, CRP und B2M; und
(ii) Anti-ECPKA-Antikörper, Anti-TK1-Antikörper, Anti-Granulin-Antikörper, Anti-Her-2-Antikörper, Anti-hP53-Antikörper, Anti-c-myc-Antikörper, cP53, hPKA, cTK1, AFP, CEA, PSA, CRP, B2M und NGAL.

4. Zusammensetzung zum Diagnostizieren von Magenkrebs nach Anspruch 3, wobei die Biomarkerkombination ferner eine Biomarkerkombination umfasst, die aus der Gruppe ausgewählt ist, die aus Folgendem besteht:
(i) Anti-ECPKA-Antikörper, Anti-TK1-Antikörper, Anti-Granulin-Antikörper, Anti-Her-2-Antikörper, Anti-hP53-Antikörper, Anti-c-myc-Antikörper, cP53 und hPKA; und
(ii) Anti-ECPKA-Antikörper, Anti-TK1-Antikörper, Anti-Granulin-Antikörper, Anti-Her-2-Antikörper, Anti-hP53-Antikörper, Anti-c-myc-Antikörper, cP53, hPKA, cTK1 und AFP.

5. Zusammensetzung zum Diagnostizieren von Magenkrebs nach Anspruch 3 oder 4, wobei die Biomarkerkombination ferner eine Biomarkerkombination umfasst, die aus der Gruppe ausgewählt ist, die aus Folgendem besteht:
(i) Anti-ECPKA-Antikörper, Anti-TK1-Antikörper, Anti-Granulin-Antikörper, Anti-Her-2-Antikörper, Anti-hP53-Antikörper, Anti-c-myc-Antikörper, cP53, hPKA und cTK1;
(ii) Anti-ECPKA-Antikörper, Anti-TK1-Antikörper, Anti-Granulin-Antikörper, Anti-Her-2-Antikörper, Anti-hP53-Antikörper, Anti-c-myc-Antikörper, cP53, hPKA, cTK1, AFP und CEA;
(iii) Anti-ECPKA-Antikörper, Anti-TK1-Antikörper, Anti-Granulin-Antikörper, Anti-Her-2-Antikörper, Anti-hP53-Antikörper, Anti-c-myc-Antikörper, cP53, hPKA, cTK1, AFP, CEA und PSA;
(iv) Anti-ECPKA-Antikörper, Anti-TK1-Antikörper, Anti-Granulin-Antikörper, Anti-Her-2-Antikörper, Anti-hP53-Antikörper, Anti-c-myc-Antikörper, cP53, hPKA, cTK1, AFP, CEA, PSA und CRP;
(v) Anti-ECPKA-Antikörper, Anti-TK1-Antikörper, Anti-Granulin-Antikörper, Anti-Her-2-Antikörper, Anti-hP53-Antikörper, Anti-c-myc-Antikörper, cTK1, AFP, CEA, CRP, B2M und NGAL;
(vi) Anti-ECPKA-Antikörper, Anti-TK1-Antikörper, Anti-Granulin-Antikörper, Anti-Her-2-Antikörper, Anti-hP53-Antikörper, Anti-c-myc-Antikörper, cTK1 und AFP;
(vii) Anti-ECPKA-Antikörper, Anti-TK1-Antikörper, Anti-Granulin-Antikörper, Anti-Her-2-Antikörper, Anti-hP53-Antikörper, Anti-c-myc-Antikörper, cTK1, AFP, CEA, PSA, CRP und B2M;
(viii) Anti-ECPKA-Antikörper, Anti-TK1-Antikörper, Anti-Granulin-Antikörper, Anti-Her-2-Antikörper, Anti-hP53-Antikörper, Anti-c-myc-Antikörper, cTKl, AFP, CEA, PSA, CRP, B2M und NGAL;
(ix) Anti-ECPKA-Antikörper, Anti-TK1-Antikörper, Anti-Granulin-Antikörper, Anti-Her-2-Antikörper, Anti-hP53-Antikörper, Anti-c-myc-Antikörper, cTK1, AFP, CEA, CRP, B2M und NGAL; und
(x) Anti-ECPKA-Antikörper, Anti-TK1-Antikörper, Anti-Granulin-Antikörper, Anti-Her-2-Antikörper, Anti-hP53-Antikörper, Anti-c-myc-Antikörper, AFP und TK1.

6. Zusammensetzung nach Anspruch 1, wobei das Expressionsniveau im Vergleich zu dem Expressionsniveau einer normalen Kontrollgruppe erhöht ist.

7. Zusammensetzung nach Anspruch 1, wobei der Antikörper ein polyklonaler Antikörper oder ein monoklonaler Antikörper ist.

8. Zusammensetzung nach Anspruch 1, wobei die Nukleinsäure ein Primer, eine Sonde, ein Antisense-Oligonukleotid oder ein Aptamer ist.

9. Kit zum Diagnostizieren von Magenkrebs, umfassend:
erste Mittel zum Messen der Expressionsniveaus von Anti-ECPKA-Antikörper, Anti-TK1-Antikörper, Anti-Granulin-Antikörper und Anti-Her-2-Antikörper; und
ein zweites Mittel zum Messen der Expressionsniveaus eines oder mehrerer Biomarker, ausgewählt aus der Gruppe bestehend aus Anti-hP53-Antikörper, Anti-c-myc-Antikörper, AFP und cTK1,
wobei die ersten Mittel oder das zweite Mittel Folgendes ist:
ein Antikörper oder ein Antigenbindungsfragment davon oder ein Aptamer, das spezifisch an das Protein bindet; oder
eine Nukleinsäure, die ein Polynukleotid umfasst, das identisch mit oder komplementär zu einem Polynukleotid ist, das das Protein kodiert.

10. Verfahren zum Detektieren eines Biomarkers zum Diagnostizieren von Magenkrebs, umfassend die folgenden Schritte:
Messen der Expressionsniveaus von (i) Anti-ECPKA-Antikörper, Anti-TK1-Antikörper, Anti-Granulin-Antikörper und Anti-Her-2-Antikörper; und (ii) einem oder mehreren Biomarkern, ausgewählt aus der Gruppe bestehend aus Anti-hP53-Antikörper, Anti-c-myc-Antikörper, AFP und cTK1, in einer biologischen Probe, die von einem Subjekt isoliert wurde, bei dem ein Verdacht auf Magenkrebs besteht; und
Vergleichen der gemessenen Expressionsniveaus mit den Expressionsniveaus in einer normalen Kontrollgruppe.

11. Verfahren nach Anspruch 10, wobei die biologische Probe Blut, Plasma, Serum, Knochenmarkflüssigkeit, Lymphflüssigkeit, Speichel, Tränenflüssigkeit, Schleimhautflüssigkeit, Fruchtwasser oder eine Kombination davon ist.

12. Verfahren nach Anspruch 10, wobei der Schritt des Messens den Schritt eines Inkubierens der biologischen Probe mit einem Antikörper oder einem Antigenbindungsfragment davon oder einem Aptamer, das spezifisch an das Protein oder Fragment davon bindet, oder eines Inkubierens eines Polynukleotids, das identisch mit oder komplementär zu einem Polynukleotid ist, das das Gen kodiert, umfasst.

13. Verfahren nach Anspruch 10, wobei der Schritt des Messens durch Elektrophorese, Immunblotten, Enzyme-linked Immunosorbent Assay (ELISA), Polymerasekettenreaktion, Northern Blotting, Polymeraseamplifikationsreaktion (Polymerasekettenreaktion: PCR), Proteinchip, Immunpräzipitation, Microarray, Elektronenmikroskopie oder einer Kombination davon durchgeführt wird.

## Revendications

1. Composition pour le diagnostic du cancer gastrique, comprenant :
des agents pour mesurer les niveaux d'expression (i) d'un anticorps anti-ECPKA, d'un anticorps anti-TK1, d'un anticorps anti-granuline et d'un anticorps anti-Her-2 ; et (ii) d'un ou plusieurs biomarqueurs choisis dans le groupe constitué par un anticorps anti-hP53, un anticorps anti-c-myc, AFP et cTK1,
dans laquelle les agents sont
un anticorps ou un fragment de liaison à un antigène de celui-ci ou un aptamère qui se lie spécifiquement à la protéine ; ou
un acide nucléique comprenant un polynucléotide identique ou complémentaire à un polynucléotide qui code la protéine.

2. Composition pour le diagnostic du cancer gastrique selon la revendication 1, dans laquelle la combinaison de biomarqueurs comprend en outre un ou plusieurs biomarqueurs choisis dans le groupe constitué par cP53, hPKA, CEA, PSA, CRP, B2M et NGAL.

3. Composition pour le diagnostic du cancer gastrique selon la revendication 1, dans laquelle la combinaison de biomarqueurs comprend une combinaison de biomarqueurs choisie dans le groupe constitué par :
(i) un anticorps anti-ECPKA, un anticorps anti-TK1, un anticorps anti-granuline, un anticorps anti-Her-2, un anticorps anti-hP53, un anticorps anti-c-myc, cP53, hPKA, cTK1, AFP, CEA, PSA, CRP et B2M ; et
(ii) un anticorps anti-ECPKA, un anticorps anti-TK1, un anticorps anti-granuline, un anticorps anti-Her-2, un anticorps anti-hP53, un anticorps anti-c-myc, cP53, hPKA, cTK1, AFP, CEA, PSA, CRP, B2M et NGAL.

4. Composition pour le diagnostic du cancer gastrique selon la revendication 3, dans laquelle la combinaison de biomarqueurs comprend en outre une combinaison de biomarqueurs choisie dans le groupe constitué par :
(i) un anticorps anti-ECPKA, un anticorps anti-TK1, un anticorps anti-granuline, un anticorps anti-Her-2, un anticorps anti-hP53, un anticorps anti-c-myc, cP53 et hPKA ; et
(ii) un anticorps anti-ECPKA, un anticorps anti-TK1, un anticorps anti-granuline, un anticorps anti-Her-2, un anticorps anti-hP53, un anticorps anti-c-myc, cP53, hPKA, cTK1 et AFP.

5. Composition pour le diagnostic du cancer gastrique selon la revendication 3 ou 4, dans laquelle la combinaison de biomarqueurs comprend en outre une combinaison de biomarqueurs choisie dans le groupe constitué par :
(i) un anticorps anti-ECPKA, un anticorps anti-TK1, un anticorps anti-granuline, un anticorps anti-Her-2, un anticorps anti-hP53, un anticorps anti-c-myc, cP53, hPKA et cTK1 ;
(ii) un anticorps anti-ECPKA, un anticorps anti-TK1, un anticorps anti-granuline, un anticorps anti-Her-2, un anticorps anti-hP53, un anticorps anti-c-myc, cP53, hPKA, cTK1, AFP et CEA ;
(iii) un anticorps anti-ECPKA, un anticorps anti-TK1, un anticorps anti-granuline, un anticorps anti-Her-2, un anticorps anti-hP53, un anticorps anti-c-myc, cP53, hPKA, cTK1, AFP, CEA et PSA ;
(iv) un anticorps anti-ECPKA, un anticorps anti-TK1, un anticorps anti-granuline, un anticorps anti-Her-2, un anticorps anti-hP53, un anticorps anti-c-myc, cP53, hPKA, cTK1, AFP, CEA, PSA et CRP ;
(v) un anticorps anti-ECPKA, un anticorps anti-TK1, un anticorps anti-granuline, un anticorps anti-Her-2, un anticorps anti-hP53, un anticorps anti-c-myc, cTK1, AFP, CEA, CRP, B2M et NGAL ;
(vi) un anticorps anti-ECPKA, un anticorps anti-TK1, un anticorps anti-granuline, un anticorps anti-Her-2, un anticorps anti-hP53, un anticorps anti-c-myc, cTK1 et AFP ;
(vii) un anticorps anti-ECPKA, un anticorps anti-TK1, un anticorps anti-granuline, un anticorps anti-Her-2, un anticorps anti-hP53, un anticorps anti-c-myc, cTK1, AFP, CEA, PSA, CRP et B2M ;
(viii) un anticorps anti-ECPKA, un anticorps anti-TK1, un anticorps anti-granuline, un anticorps anti-Her-2, un anticorps anti-hP53, un anticorps anti-c-myc, cTK1, AFP, CEA, PSA, CRP, B2M et NGAL ;
(ix) un anticorps anti-ECPKA, un anticorps anti-TK1, un anticorps anti-granuline, un anticorps anti-Her-2, un anticorps anti-hP53, un anticorps anti-c-myc, cTK1, AFP, CEA, CRP, B2M et NGAL ; et
(x) un anticorps anti-ECPKA, un anticorps anti-TK1, un anticorps anti-granuline, un anticorps anti-Her-2, un anticorps anti-hP53, un anticorps anti-c-myc, AFP et TK1.

6. Composition selon la revendication 1, dans laquelle le niveau d'expression est augmenté par rapport au niveau d'expression d'un groupe témoin normal.

7. Composition selon la revendication 1, dans laquelle l'anticorps est un anticorps polyclonal ou un anticorps monoclonal.

8. Composition selon la revendication 1, dans laquelle l'acide nucléique est une amorce, une sonde, un oligonucléotide antisens ou un aptamère.

9. Trousse pour le diagnostic du cancer gastrique, comprenant :
des premiers agents pour mesurer les niveaux d'expression d'un anticorps anti-ECPKA, d'un anticorps anti-TK1, d'un anticorps anti-granuline et d'un anticorps anti-Her-2 ; et
un second agent pour mesurer les niveaux d'expression d'un ou plusieurs biomarqueurs choisis dans le groupe constitué par un anticorps anti-hP53, un anticorps anti-c-myc, AFP et cTK1,
dans laquelle les premiers agents ou le second agent sont
un anticorps ou un fragment de liaison à un antigène de celui-ci ou un aptamère qui se lie spécifiquement à la protéine ; ou
un acide nucléique comprenant un polynucléotide identique ou complémentaire à un polynucléotide qui code la protéine.

10. Procédé pour la détection d'un biomarqueur pour le diagnostic du cancer gastrique, comprenant les étapes de :
mesure des niveaux d'expression (i) d'un anticorps anti-ECPKA, d'un anticorps anti-TK1, d'un anticorps anti-granuline et d'un anticorps anti-Her-2 ; et (ii) d'un ou plusieurs biomarqueurs choisis dans le groupe constitué par un anticorps anti-hP53, un anticorps anti-c-myc, AFP et cTK1 dans un échantillon biologique isolé d'un sujet soupçonné d'avoir un cancer gastrique ; et
comparaison des niveaux d'expression mesurés aux niveaux d'expression dans un groupe témoin normal.

11. Procédé selon la revendication 10, dans lequel l'échantillon biologique est du sang, du plasma, du sérum, du liquide de moelle osseuse, du liquide lymphatique, de la salive, du liquide lacrymal, du liquide des muqueuses, du liquide amniotique ou une combinaison de ceux-ci.

12. Procédé selon la revendication 10, dans lequel l'étape de mesure comprend l'étape d'incubation de l'échantillon biologique avec un anticorps ou un fragment de liaison à un antigène de celui-ci ou un aptamère qui se lie spécifiquement à la protéine ou à un fragment de celle-ci, ou d'incubation d'un polynucléotide identique ou complémentaire à un polynucléotide qui code le gène.

13. Procédé selon la revendication 10, dans lequel l'étape de mesure est réalisée par électrophorèse, immunobuvardage, essai immuno-enzymatique (ELISA), réaction en chaîne par polymérase, buvardage de Northern, amplification par polymérase (réaction en chaîne par polymérase : PCR), puce à protéines, immunoprécipitation, puce à ADN, microscopie électronique, ou une combinaison de ceux-ci.
